# FASCICULE DE BREVET EUROPEEN

(11) **EP 0 306 422 B1**
(45) Date de publication et mention de la délivrance du brevet: **29.05.1996**
(21) Numéro de dépôt: 88420298.7
(22) Date de dépôt: 01.09.1988
(51) Int. Cl.: A61K 31/135, A61K 9/18

(54) **Nouvelles formes galéniques de béta-2-mimétiques pour administration par voie per- et sublinguale**
Galenische 2-beta-Mimetik-Formen für die per- und sublinguale Verabreichung
Galenical forms of 2-beta-mimetics for perlingual and sublingual administration

(30) Priorité: 02.09.1987 FR 8712708
(43) Date de publication de la demande: 08.03.1989
(73) Titulaire: MEDIBREVEX SA, 74940 Annecy Le Vieux (FR)
(72) Inventeur: Ayache Clairet, Josiane, F-38000 Grenoble (FR); Ayache, Jean-Jacques, F-38000 Grenoble (FR); Bruttmann, Georges, F-38000 Grenoble (FR); Pedrali, Patrick, F-74000 Annecy (FR); Robert, Serge, B-1440 Braine le Chateau (BE)
(74) Mandataire: Maureau, Philippe

(56) Documents cités:
- EP-A- 0 205 282
- CH-A- 156 609
- FR-A- 2 285 896
- M. NETIEN et al.: "Galenica", vol. 16, "Médicaments Homéopathiques", édition 2, 1986, pages 77-99, chapitre 2, Technique et Documentation, Paris, FR
- Remington's Pharmaceutical Sciences, 15th edition, 1975, pages 684 et 1577

## Description

La présente invention concerne de nouvelles formes galéniques de béta-2-mimétiques pour administration par voie per- et sublinguale, et l'utilisation de ces composés pour la préparation de telles formes pour le traitement des affections respiratoires dyspnéisantes.

On connaît toute l'importance que présente l'utilisation des bronchodilatateurs dans certaines affections respiratoires dyspnéisantes telles que l'asthme, la bronchite chronique et l'emphysème.

Les béta-2-mimétiques tiennent une place importante parmi les bronchodilatateurs.

C'est ALQUIST qui, en 1948, découvrit l'existence de récepteurs qui réagissent differemment aux cathécholamines : il décrivit des récepteurs alpha, vecteurs des réactions excitatrices et des récepteurs béta, vecteurs des réactions inhibitrices.

En 1967, LANDS différencia ces récepteurs en béta 1 et béta 2, les béta 1 servant à la stimulation du coeur et des tissus graisseux, les béta 2 au relâchement des muscles lisses bronchiques, vasculaires et utérins.

A la suite de cette découverte, des médicaments sélectifs béta 2 (donc spécifiques des récepteurs du même nom) ont été mis sur le marché et leur indication principale a été l'asthme.

L'activité broncho-dilatatrice des produits est mise en évidence par l'amélioration du volume expiratoire maximum seconde (V.E.M.S. ou F.E.V.I. des anglo-saxons) enregistré par spirographie.

L'activité clinique et l'utilité des béta-2-mimétiques ne sont plus à démontrer.

Les béta-2-mimétiques existent actuellement sous diverses formes galéniques permettant leur administration :
- par voie orale : sirops et comprimés,
- par voie injectable,
- par aérosols doseurs.

Conformément au document EP-A-0 205 282, on a décrit des compositions thérapeutiques constituées chacune par un composé Béta-2-mimétique, par exemple du salbutamol, sur un support solide, pharmaceutiquement acceptable, par exemple un comprimé, adapté à une forme d'administration principalement orale, c'est-à-dire avec déglutition ou ingestion, et accessoirement buccale, c'est-à-dire au travers de la muqueuse buccale du patient.

Les béta-2-mimétiques sont actifs par voie orale, mais leurs effets n'apparaissent qu'environ deux heures après leur administration. De plus, il existe une inactivation dans le tube digestif et le foie.

Par voie injectable (sous-cutanée ou intra-veineuse), les effets sont observés rapidement.

L'utilisation sous forme d'aérosols est sans doute la meilleure, à condition que les voies aériennes ne soients pas encombrées et que l'utilisateur de l'aérosol doseur par le malade soit la plus parfaite possible. Or, peu de patients utilisent correctement les appareils mis à leur disposition, ce qui aboutit fréquemment à un abus médicamenteux, car le malade augmente la dose, ayant l'impression qu'une bouffée ou deux sont inefficaces. Par ailleurs, il n'est pas certain qu'une bouffée d'aérosol atteigne les voies périphériques. Comme cela a déjà été souligné chez les patients présentant une maladie asthmatique en poussée aiguë avec obstruction bronchique (aggravée par une infection ou par une hypersécrétion bronchique), il est hasardeux d'attendre des effets bénéfiques de cet aérosol.

Les inventeurs ont découvert une nouvelle forme médicamenteuse des composés Béta-2-mimétiques comprenant un support solide divisé délitable sous l'action de la salive et adapté spécifiquement à une administration sublinguale, cette forme étant différente des formes retard décrites dans le document EP-A-0 205 282, en ce qu'elle comprend un support solide ne comprenant pas de comprimés et enrobés d'une cellulose adhérant aux muqueuses de la bouche.

Cette voie per- et sublinguale est utilisée à cause de sa configuration anatomique qui en fait une entité par rapport aux autres éléments de la cavité buccale.

Schématiquement, on peut décrire la région sublinguale de la manière suivante (chacun des éléments constitutifs ayant des propriétés utiles dans l'administration des médicaments introduits par cette voie).

Globalement, la région sublinguale comprend :
- la face inférieure de la langue qui sert de plafond, très riche en vaisseaux sanguins : veines, artères et vaisseaux lymphatiques ;
- le plancher de la bouche qui constitue la partie inférieure de la région sublinguale. C'est également une région anatomique qui comporte beaucoup de vaisseaux sanguins et surtout un très riche réseau veineux, des artères et des vaisseaux lymphatiques ;
- les bords de la région sublinguale sont formés par les parties montantes du maxillaire inférieur, les gencives et les dents.

Dans le plancher de la bouche, on trouve des glandes salivaires, les glandes sublinguales, qui secrètent de la salive dès qu'il existe un contact avec la région sublinguale. Par sa composition, la salive participe activement au délitement du produit pharmacologiquement actif présenté sous cette forme galénique sublinguale particulière selon l'invention.

Par ailleurs, la structure histologique montre l'existence de cellules immuno-compétentes en grand nombre qui vont, dans certains cas, favoriser l'activité médicamenteuse.

Par rapport aux autres constituants de la bouche : intérieur des joues, partie supérieure de la langue, la région sublinguale apparaît donc comme une entité anatomique particulière qui en fait une voie d'administration médicamenteuse privilégiée comparable à l'administration des médicaments par voie injectable.

Avec un médicament selon l'invention, l'activité du produit est fondée sur sa possibilité d'absorption rapide sans déglutition.

Conformément au document :
Galenica 16 : médicaments homéopathiques, 2è édition, 1986 on a décrit en soi un procédé d'obtention d'un médicament, comparable à celui défini précédemment, mais pour une spécialité homéopathique. Un tel procédé appliqué à l'homéopathie aboutit à un médicament pour lequel le principe actif n'est plus dosable, donc contrôlable, soit parce que la quantité théoriquement présente sur le support actif est trop faible par rapport au seuil de la sensibilité des méthodes traditionnelles d'analyse, soit parce que cette quantité n'existe plus du tout, compte tenu de la dilution utilisée.

Les avantages des médicaments selon l'invention sont nombreux :
- le passage sublingual est rapide : il ne nécessite ni aérosol doseur, ni matériel d'injection ; bien entendu, cette forme galénique n'est indiquée que pour les traitements bronchodilatateurs des malades "en ambulatoire" et, en aucun cas, pour les malades dont l'état clinique relève d'une hospitalisation ou d'un asthme très grave ;
- la présentation selon l'invention permet d'avoir une posologie rigoureuse sans risque d'abus (les doses prescrites sont limitées par une ordonnance) ;
- l'efficacité est conservée, même s'il y a encombrement bronchique, puisque le passage se fait par le réseau vasculaire sub- et perlingual.

Le procédé d'obtention des nouvelles formes galéniques selon l'invention va être exposé en détail.

Le produit de départ est le sulfate de salbutamol que l'on peut obtenir à la concentration de 0,1 mg par ml par exemple, voire à d'autres concentrations.

Le produit de départ est lyophilisé.

Le lyophilisat obtenu est mis en solution dans tout solvant convenable, de façon à obtenir une solution mère. Le solvant utilisé peut être l'eau ou le sérum physiologique ; il peut également être choisi parmi d'autres solvants, de préférence polaires, tel que l'alcool éthylique de titre faible.

En cas de dilutions de la solution mère, toutes les précautions sont prises pour que la quantité de solvant reste constante, ceci afin de réaliser toutes les imprégnations de façon identique.

On réalise ensuite, dans une turbine, à l'aide d'un injecteur de type dénommé "spray-doseur", l'imprégnation, à l'aide de chacune des dilutions, de globules constitués de façon connue en soi, d'un support solide pharmaceutiquement acceptable et spécialement d'un mélange saccharose-lactose. Il est évident que, sans sortir du cadre de l'invention, ces globules pourraient être remplacés par des poudres ou des comprimés, également adaptés à l'application par voie perlinguale ou sublinguale.

La technique d'imprégnation est celle de la multi-imprégnation ou de l'imprégnation fractionnée de manière à garantir une parfaite répartition du principe actif homogène.

Entre chaque imprégnation, le séchage est réalisé à une température inférieure ou égale à 30°C par passage d'air forcé et desséché dans une chambre où règne une dépression obtenue par la différence du débit existant entre ce passage d'air et un puissant extracteur.

La dernière imprégnation est une imprégnation protectrice du type "dragéification".

Afin de garantir que soit totalement maintenue l'intégrité physico-chimique du principe actif, les différentes étapes de l'imprégnation sont avantageusement effectuées sous atmosphère d'azote.

La dernière opération consiste à mettre en gélules les globules ainsi obtenus, ou éventuellement à placer lesdits globules ou les poudres ou comprimés dans des tubes-doses et à terminer le conditionnement de façon classique (blisters pour les gélules, coffrets pour les tubes-doses).

Différents types de traitement peuvent être envisagés :
- traitement quotidien : prise de deux à quatre doses perlinguales à adapter selon la spirographie et les symptomes cliniques ;
- traitement occasionnel : prise de deux à quatre capsules lors d'une crise, prise que l'on pourra répéter jusqu'à la "récupération" respiratoire.

Les résultats cliniques sont très surprenants par rapport aux aérosols et aux formes orales.

Un autre avantage tout à fait surprenant est la rapidité d'action des nouvelles formes galéniques selon l'invention qui vont permettre au malade de réduire rapidement la posologie quotidienne ; le malade n'a plus besoin d'actes infirmiers (injections sous-cutanées). Seuls restent nécessaires les contrôles médicaux qui seront dictés par l'état respiratoire du malade.

Il est bien évident que la présente invention ne saurait se limiter aux concentrations, modes de fractionnement ou dilutions donnés ci-avant à titre d'exemple non limitatif ; ces concentrations peuvent, bien entendu, être adaptées sur demande ; de même le support saccharose-lactose peut être remplacé par un autre support pharmaceutiquement acceptable adapté.

## Revendications (Revendications pour l'(les) Etat(s) contractant(s) suivant(s): AT, BE, CH, LI, DE, FR, GB, IT, LU, NL, SE)

1. Composition thérapeutique comprenant un composé Béta-2-mimétique, par exemple du salbutamol, sur un support solide, pharmaceutiquement acceptable, sous une forme d'administration buccale, caractérisée en ce que le support solide est divisé, délitable sous l'action de la salive, et adapté spécifiquement à une administration sublinguale, à l'exclusion de tout support solide comprenant des comprimés et enrobés d'une cellulose adhérant aux muqueuses de la bouche.

2. Composition selon la revendication 1, caractérisée en ce que le support solide comprend un mélange de saccharose et de lactose.

## Revendications (Revendications pour l'(les) Etat(s) contractant(s) suivant(s): ES, GR)

1. Procédé de préparation d'une composition thérapeutique, selon lequel on dispose un composé Béta-2-mimétique, par exemple du salbutamol, sur un support solide, pharmaceutiquement acceptable, sous une forme d'administration buccale, caractérisé en ce qu'on divise le support solide, sous une forme délitable sous l'action de la salive, et adapté spécifiquement à une administration sublinguale, mais excluant les comprimés et enrobés d'une cellulose adhérant aux muqueuses de la bouche.

2. Procédé selon la revendication 1, caractérisé en ce qu'on mélange du saccharose et du lactose pour obtenir un support solide.

## Claims (Claims for the following Contracting State(s): AT, BE, CH, LI, DE, FR, GB, IT, LU, NL, SE)

1. Therapeutic composition comprising a beta₂-mimetic compound, for example salbutamol, on a pharmaceutically acceptable solid carrier, in a form for buccal administration, characterized in that the solid carrier is divided, can disintegrate through the action of saliva and is specifically suited to sublingual administration, excluding any solid carrier which comprises tablets and which are [sic] coated with a cellulose adhering to the mucosae of the mouth.

2. Composition according to Claim 1, characterized in that the solid carrier comprises a mixture of sucrose and lactose.

## Claims (Claims for the following Contracting State(s): ES, GR)

1. Process for preparing a therapeutic composition, according to which a beta₂-mimetic compound, for example salbutamol, is arranged on a pharmaceutically acceptable solid carrier, in a form for buccal administration, characterized in that the solid carrier, in a form which can disintegrate through the action of saliva, is divided, and is specifically suited to sublingual administration, but excluding tablets and which are [sic] coated with a cellulose adhering to the mucosae of the mouth.

2. Process according to Claim 1, characterized in that sucrose and lactose are mixed to obtain a solid carrier.

## Patentansprüche (Patentansprüche für folgende(n) Vertragsstaat(en): AT, BE, CH, LI, DE, FR, GB, IT, LU, NL, SE)

1. Therapeutische Zusammensetzung, enthaltend ein Beta-2-Mimetikum, bspw. Salbutamol, auf einem pharmazeutisch verträglichen festen Träger in einer oral verabreichbaren Form, dadurch gekennzeichnet, daß der Träger teilig ist, sowie durch die Wirkung von Speichel zerfällt, und daß dieser speziell für eine sublinguale Verabreichung angepaßt ist, mit der Ausnahme von jeglichen festen Trägern, die Tabletten aus Cellulose und Cellulosebeschichtungen, welche an der Mundschleimhaut haften, umfassen.

2. Zusammensetzung nach Anspruch 1, dadurch gekennzeichnet, daß der Träger eine Mischung von Saccharose und Lactose enthält.

## Patentansprüche (Patentansprüche für folgende(n) Vertragsstaat(en): ES, GR)

1. Verfahren zum Herstellen einer therapeutischen Zusammensetzung, bei dem ein Beta-2-Mimetikum, bspw. Salbutamol, auf einen pharmazeutisch verträglichen festen Träger in einer oral verabreichbaren Form gebracht wird, dadurch gekennzeichnet, daß der feste Träger teilig gemacht wird, und zwar in eine Form, die durch die Wirkung von Speichel zerfällt, und daß dieser speziell für eine sublinguale Verabreichung angepaßt ist, wobei jedoch Tabletten aus Cellulose und Cellulosebeschichtungen, die an der Mundschleimhaut haften, ausgenommen sind.

2. Verfahren nach Anspruch 1, dadurch gekennzeichnet, daß man Saccharose und Lactose mischt, um einen festen Träger zu erhalten.
